# EUROPEAN PATENT APPLICATION

(11) **EP 3 315 098 A1**
(43) Date of publication of application: **02.05.2018**
(21) Application number: 17198182.2
(22) Date of filing: 25.10.2017
(51) Int. Cl.: A61F 2/50, A61F 5/058

(54) **FRACTURE BRACES**

(30) Priority: 25.10.2016 BE 165801
(71) Applicant: Université Libre de Bruxelles, 1050 Bruxelles (BE)
(72) Inventor: SCHUIND, Frédéric, 1600 Sint-Pieters-Leeuw (BE)
(74) Representative: De Clercq & Partners

(57) **Abstract**

Provided herein are patient-specific fracture braces and methods for the design thereof. The methods of designing a brace according to the present invention are based on the symmetry between opposite limbs (3), and involve designing a patient-specific brace for a fractured limb based on a model of the non-fractured contralateral limb.

## Description

### FIELD OF THE INVENTION

The present invention relates to fracture braces and methods for the design and production thereof. The braces are particularly useful for the treatment of fragility fractures.

### BACKGROUND OF THE INVENTION

In the last years, there has been a marked increase in the incidence of fragility fractures. Fragility fractures occur as a result of normal activities, such as a fall from standing height or less, and typically occur in osteoporotic patients. Although these fractures usually are relatively simple, they exhibit a low stability. Indeed, the fractures often require osteosynthesis due to redisplacement under plaster cast immobilization, despite an initial acceptable reduction.

The problem of fragility fractures concerns older patients but also relatively young osteoporotic patients. Geriatric patients with poor bone quality in particular suffer in the pre- and post-operative periods, and often suffer from complications. In addition to the patient's suffering, fragility fractures result in significant healthcare costs, such as the costs of orthopaedic implants and the costs of surgical operations.

Redisplacement of the fracture under non-operative treatment is often related to the fact that plaster casts are initially applied on a swollen limb. The cast cannot be applied too tight, as this cannot be tolerated by the patient. When the swelling subsides after a few days, the fracture often is no longer adequately contained by the cast. Moreover, Computer Tomography imaging has shown also that conventional plaster casts frequently are shaped irregularly, thereby offering poor contention.

As an alternative for rigid plaster casts, an adjustable fracture brace may be used. Such braces can be tightened progressively on a fractured limb, thereby avoiding fracture redisplacement. International patent application WO2013/028633 describes a custom made adjustable brace for supporting a fractured limb, wherein the inner portion of the brace corresponds to a digital representation of the fractured limb. The brace is designed to closely fit the fractured limb when it is initially injured. When the swelling of the limb reduces, the brace can be tightened around the limb. The fracture must be reduced before the digital representation of the limb is generated. If the reduction is not performed properly, the brace does not provide the support needed for optimal healing.

It is an aim of the present invention to provide braces and methods for the design and production thereof, which mitigate at least one of the problems stated above.

### SUMMARY OF THE INVENTION

The present invention relates to fracture braces and methods for the design and production thereof. The braces are particularly useful for the treatment of fragility fractures. The methods of designing a brace according to the present invention are based on the symmetry between opposite limbs, and involve designing a patient-specific brace for a fractured limb based on a model of the contralateral limb which is not fractured.

Accordingly, in a first aspect, provided herein is a method of designing a patient-specific brace for positioning on a fractured limb, based on a three-dimensional (3D) model and/or morphological data of at least a part of a contralateral (i.e. not fractured) limb. More particularly, provided herein are the following aspects:
Aspect 1. A method of designing a patient-specific brace for positioning on a fractured limb, said method comprising:
   (a) providing a 3D model and/or morphological data of at least a part of a contralateral (i.e. not fractured) limb; and
   (b) designing a patient-specific brace for said fractured limb, based on said 3D model and/or morphological data provided in step (a) and assuming symmetry between said fractured limb and said contralateral limb, wherein said brace comprises first and second portions having an adjustable position relative to each other so as to adjust the internal dimensions of said brace.
Aspect 2. The method according to aspect 1, wherein the interior of said brace is provided with a patient-specific contact surface which is the mirror image of a corresponding part of the outer surface of said 3D model.
Aspect 3. The method according to aspect 1 or 2, wherein said first and second portions are coupled to each other via one or more straps.
Aspect 4. The method according to aspect 3, wherein said one or more straps are elastic.
Aspect 5. The method according to any one of aspects 1 to 4, wherein said brace comprises a casing which is at least partially made of a resilient material so as to adjust the internal dimensions of said casing.
Aspect 6. The method according to any one of aspects 1 to 5, wherein the inner surface of said brace is provided with one or more inflatable cushions.
Aspect 7. The method according to any one of aspects 1 to 6, wherein the inner surface of said brace is provided with one or more resilient pads.
Aspect 8. The method according to any one of aspects 1 to 7, wherein said morphological data comprises the length and the perimeter of said contralateral limb.
Aspect 9. The method according to any one of aspects 1 to 8, wherein said brace further comprises one or more pressure sensors.
Aspect 10. The method according to any one of aspects 1 to 9, wherein the fracture of said fractured limb is a wrist fracture or an ankle fracture.
Aspect 11. The method according to any one of aspects 1 to 10, further comprising the step of: (c) manufacturing said brace at least partially via additive manufacturing.
Aspect 12. In a further aspect, provided herein is a patient-specific brace obtained by the method according to any one of aspects 1 to 11.
Aspect 13. In a further aspect, the present application provides a patient-specific brace configured to fit a fractured limb, wrist or ankle comprising first and second portions having an adjustable position relative to each other so as to adjust the internal dimensions of said brace, characterized in that said patient-specific brace is designed based on the morphology of the contralateral limb, wrist or ankle.
Aspect 14. The patient-specific brace according to aspect 13, wherein said first and second portions are coupled to each other via one or more straps.
Aspect 15. The patient-specific brace according to aspects 13 or 14, wherein said one or more straps are elastic.
Aspect 16. The patient-specific brace according to any one of aspects 13 to 15, wherein said brace comprises a casing which is at least partially made of a resilient material so as to adjust the internal dimensions of said casing.
Aspect 17. The patient-specific brace according to any one of aspects 13 to 16, wherein the inner surface of said brace is provided with one or more inflatable cushions.
Aspect 18. The patient-specific brace according to any one of aspects 13 to 17, wherein the inner surface of said brace is provided with one or more resilient pads. Aspect 19. In a further aspect, the present application provides a computer program on a computer-readable storage medium, wherein said program is configured for carrying out the method according to any one of aspects 1 to 11.
Aspect 20. In yet a further aspect, the present application provides a method of treating a fracture of a fractured limb, said method comprising:
   (i) optionally performing a preliminary reduction of said fracture;
   (ii) providing a 3D model and/or morphological data of the contralateral (i.e. non-fractured) limb;
   (iii) providing a patient-specific brace for treating said fracture, wherein said brace is designed based on said 3D model and/or morphological data provided in step (ii);
   (iv) optionally performing further reduction of said fracture; and
   (v) applying said patient-specific brace to said fractured limb.
Aspect 21. The method according to aspect 20, further comprising: (vi)progressive tightening of said brace upon a decrease of swelling of said fractured limb to maintain pressure on said fractured limb.

The braces provided via the design method described herein can provide an improved healing of fracture, compared to standard braces or custom braces which are designed based on images of the fractured limb. An improved healing can significantly reduce the need for operative treatment, thereby saving the patient from additional suffering and reducing healthcare costs. Moreover, the methods of designing braces described herein may allow for designing custom braces before the fracture is reduced, such that the brace can be provided to the patient in a very short time. The above and other characteristics, features and advantages of the concepts described herein will become apparent from the following detailed description, which illustrates, by way of example, the principles of the invention.

### BRIEF DESCRIPTION OF THE DRAWINGS

The drawings depicted herein are merely for illustrative purposes and are not to be seen as limiting the invention in any particular way.

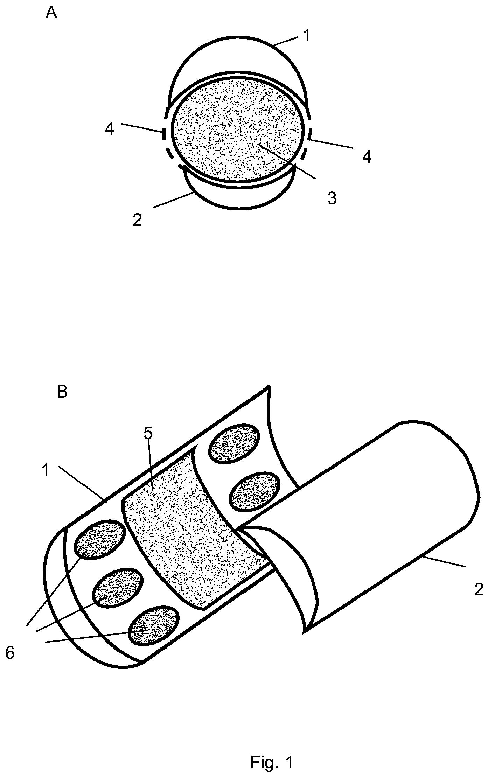

- **Fig. 1 A:**: Schematic cross-section of a first portion (1) and second portion (2) of a particular embodiment of the brace described herein applied on a limb (3), wherein the portions are connected via straps (4). **B**: Schematic illustration of a first portion (1) and second portion (2) of a particular embodiment of the brace described herein, wherein the first portion (1) is provided with a patient-specific contact surface (5) and resilient pads (6).

In the figures, the following numbering is used:
1 - first portion; 2 - second portion; 3 - limb; 4 - strap; 5 - contact surface; 6 - pad.

### DETAILED DESCRIPTION OF THE INVENTION

While potentially serving as a guide for understanding, any reference signs used herein and in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited components, elements or method steps also include embodiments which "consist of" said recited components, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments described herein are capable of operation in other sequences than described or illustrated herein.

The values as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to ensure one or more of the technical effects envisaged herein. It is to be understood that each value as used herein is itself also specifically, and preferably, disclosed. Typically, the term "about" should be read in this context.

The recitation of numerical ranges by endpoints includes all numbers and fractions subsumed within the respective ranges, as well as the recited endpoints.

All documents cited in the present specification are hereby incorporated by reference in their entirety.

Unless otherwise defined, all terms used in disclosing the concepts described herein, including technical and scientific terms, have the meaning as commonly understood by one of ordinary skill in the art. By means of further guidance, definitions for the terms used in the description are included to better appreciate the teaching of the present disclosure. The terms or definitions used herein are provided solely to aid in the understanding of the teachings provided herein.

Reference throughout this specification to "one embodiment" or "an embodiment" means that a particular feature, structure or characteristic described in connection with the embodiment is included in at least one embodiment envisaged herein. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are also envisaged herein, and form different embodiments, as would be understood by those in the art. For example, in the appended claims, any of the features of the claimed embodiments can be used in any combination. Provided herein are patient-specific braces for supporting a fractured limb, and methods for the design and manufacture thereof. The braces are particularly useful for the treatment of fragility fractures, but may also be used for the treatment of other fractures. The methods of designing a brace according to the present invention are based on the symmetry between opposite limbs, and involve designing a patient-specific brace for a fractured limb based on a model of the contralateral limb which is not fractured.

More particularly, in a first aspect, the present invention provides a method of designing a patient-specific brace for positioning on a fractured limb. The present method of designing a brace comprises:
(a) providing a three-dimensional (3D) model and/or morphological data of at least a part of a contralateral (non-fractured) limb; and
(b) designing a patient-specific brace for the fractured limb, based on the 3D model and/or morphological data provided in step (a); and assuming symmetry between the fractured limb and the contralateral limb.

The brace designed by the present method typically comprises first and second portions having an adjustable position relative to each other, so as to adjust the internal dimensions of the brace. This will be explained further herein below.

The method of designing a brace described herein, also referred herein as "design method", can be used for designing a brace for positioning on any limb on the human or vertebrate animal body wherein the contralateral limb is not fractured. The term "contralateral limb" as used herein refers to the corresponding limb of the patient on the opposite side to the fractured limb. For example, if the fractured limb is the right leg, then the contralateral limb is the patient's left leg.

In preferred embodiments, the limb is a limb of the human body, such as an arm or a leg. In specific embodiments, the fracture of the fractured limb may be a wrist fracture or an ankle fracture. However, the present method of designing braces is not limited thereto.

The present design method comprises in a first step (a) the provision of a 3D model and/or morphological data of at least a part of a contralateral (non-fractured) limb. In preferred embodiments, the 3D model is a digital model. In case of a joint fracture, the position of the contralateral limb in the 3D model typically corresponds to the optimal position for joint immobilization. The 3D model typically comprises a model of at least a part of the outer surface of the contralateral limb. The relevant parts of the contralateral limb to be included in the 3D model may depend on the location of the fracture, the regions of the fractured limb which need to be supported, and the region of the fractured limb which exhibits most swelling. In particular embodiments, the present design method involves selecting one or more support areas for supporting the fractured limb, and providing a 3D model of the contralateral limb including at least the areas symmetrical to the selected support areas.

Methods for obtaining a 3D model of a limb are known in the art. Suitable methods include but are not limited to 3D laser scanning such as triangulation-based 3D laser scanning, stereophotogrammetry, image correlation, sonography, computed tomography, and X-ray imaging.

Prior to obtaining the 3D model of the contralateral limb, the contralateral limb may be provided with one or more markings which may provide useful reference points for generating the model and/or for designing the brace. In certain embodiments, the patient's limb may be marked with a pen, with stickers, or other means of providing reference signs. In particular embodiments, the markings may indicate the edge of the brace.

In a next step (b), a patient-specific brace is designed for the fractured limb, based on the 3D model and/or morphological data obtained in step a). Indeed, the 3D model and/or morphological data of the contralateral limb allows for designing a patient-specific brace for the fractured limb, based on the symmetry between the opposite limbs. More particularly, it can be assumed that the contralateral limb essentially is the mirror image of the (properly realigned) fractured limb.

The design of the brace for the fractured limb based on the 3D model of the contralateral limb can be done either by:
- first obtaining a mirror image of the 3D model of the contralateral limb (or a part thereof), followed by designing a brace which fits the mirror image, or
- first designing a brace which fits the (3D model of the) contralateral limb; followed by obtaining a mirror image of this brace.

Both strategies result in a brace which fits the fractured limb and can provide the required support for optimal healing of the fracture.

In particular embodiments, the brace may be designed based on key anatomical features of the contralateral limb instead of a 3D model. Thus, in these embodiments, step (a) involves providing morphological data of at least a part of the contralateral limb, whereas step (b) involves designing a patient-specific brace for the fractured limb, based on the morphological data provided in step (a), assuming symmetry between the fractured limb and the contralateral limb. This may be preferred for various reasons, for example when there is no access to imaging technologies, or in emergency situations. Thus, in particular embodiments, the brace may be designed based on morphological data of the contralateral limb, such as the length of the contralateral limb, and/or the perimeter of the contralateral limb at one or more positions. These data may then be used for obtaining an appropriate design for a patient-specific brace matching the mirror image of the contralateral limb, for example via a morphologic database or another source.

In certain embodiments, the brace may be designed based on the 3D model of the contralateral limb, combined with additional morphological data of the fractured limb and/or of the contralateral limb.

The braces obtainable by the present design method comprise first and second portions, which are typically rigid objects, such that they can adequately support and immobilize the fractured limb. The first and second portions are adjustably and preferably releasably coupled to each other, thereby allowing an adjustment of the internal dimensions of the brace. In this way the brace can be adjusted to changing dimensions of the fractured brace (for example upon a decrease of the swelling of the limb), thereby providing a continuous support and (partial) immobilization of the fractured limb. In certain embodiments, the braces may be provided with a third portion and optional further portions, which may have an adjustable position relative to the first and/or second portions.

Typically, the first and second portions are applied on opposite sides of the limb, thereby providing a bivalve brace. The first and second portions typically have a concave inner surface for contacting the fractured limb. Typically, at least one of the portions has a longitudinal shape. The term "longitudinal" as used herein refers to objects having an aspect ratio (length divided by width) of at least 2. A schematic illustration of a bivalve brace as described herein is provided in Fig. 1A and B. The brace comprises a first portion (1) and a second portion (2), each having a longitudinal shape and a concave inner surface for contacting a fractured limb (3).

The coupling between the first and second portions may be obtained in various ways, for example via circular elastic bands surrounding the first and second portions, via straps, or other methods.

In particular embodiments, the first and second portions may be coupled to each other via via one or more straps. Straps can allow a releasable and adjustable coupling between the first and second portions, such that the relative position of the first and second portion can be adjusted to the (changing) dimensions of the fractured limb. In Fig. 1A, the first portion (1) and second portion (2) are connected via straps (4, dotted lines).

The straps may be elastic or inelastic (rigid). Elastic straps allow for the brace to adjust automatically to dimensional changes of the limb, without intervention of the patient or doctor. On the other hand, inelastic straps may allow for a better regulation of the pressure.

The straps may have a fixed connection to one of the first and second portions, and be connected or connectable in a releasable and/or adjustable way to the other portion, or to another location on the same portion. Releasable and adjustable connection of the straps may be obtained in a variety of ways, e.g. via a buckle, via Velcro®, or other ways.

In addition to the straps or as an alternative thereto, the brace may comprise a casing which is at least partially made of a resilient material so as to adjust the internal dimensions of the casing. The first and second portions of the brace are typically provided on the casing. For example, the first and second portions may be provided on the interior of a sleeve which is at least partially made of an elastic fabric. The casing may be provided with a hinge and/or straps for opening and closing the casing. In certain embodiments, the casing may be at least partially made of a shape memory material.

In particular embodiments, the interior of said brace is provided with a patient-specific contact surface which is the mirror image of a corresponding part of the outer surface of said 3D model and/or morphogical data described above. More particularly, the patient-specific contact surface is configured to fit onto one or more discrete areas on the mirror image of the 3D model and/or morphogical data described above. Considering the symmetry between opposed limbs, the match between the contact surface and the mirror image of the (3D model of the) contralateral limb ensures a tight fit of the brace on the fractured limb, and can ensure that the support provided by the brace favors the optimal alignment of the fractured limb. The contact surface may be a continuous or discontinuous surface. In specific embodiments, at least one of the first and second portions may be provided with a contact surface as described above. Fig. 1B shows a first portion (1) of a brace provided with a patient-specific contact surface (5) as described herein.

Typically, the patient-specific contact surface is a free-form structure fitting at least part of the outer surface of the mirror image of the 3D model of the contralateral limb. The term "free-form structure" as used herein refers to a structure having an irregular and/or asymmetrical flowing shape or contour, more particularly fitting at least a part of the outer surface of the mirror image of the 3D model of the contralateral limb.

Thus, in particular embodiments, the free-form structure is a free-form surface. A free-form surface refers to an (essentially) two-dimensional shape contained in a three-dimensional geometric space. Indeed, as will be detailed below, such a surface can be considered as essentially two-dimensional but may have a varying thickness. Typically, the free-form structure or surface is characterized by a lack of rigid radial dimensions, unlike regular surfaces such as planes, cylinders and conic surfaces. Free-form surfaces are known to the skilled person and widely used in engineering design disciplines. Typically non-uniform rational B-spline (NURBS) mathematics is used to describe the surface forms; however, there are other methods such as Gorden surfaces or Coons surfaces. The form of the free-form surfaces are characterized and defined not in terms of polynomial equations, but by their poles, degree, and number of patches (segments with spline curves). Free-form surfaces can also be defined as triangulated surfaces, where triangles are used to approximate the 3D surface. Triangulated surfaces are used in STL (Standard Triangulation Language) files that are known to a person skilled in computer aided design (CAD).

In certain embodiments, the first and second portions both are provided with one or more patient-specific contact surfaces. In other embodiments, only the first portion is provided with a patient-specific contact surface, whereas the second portion is provided with one or more standard support surface(s). For example, in the case of a fracture of the distal radius, the first portion may be provided with a patient-specific contact surface for positioning on the dorsal part of the patient's wrist.

In certain embodiments, the interior of the brace may be provided with one or more resilient pads (6), for example as shown in Fig. 1B. This may increase the brace wearer's comfort. Examples of resilient pressure pads which are suitable for use with the present braces are described in international patent application WO 95/33428, which is hereby incorporated by reference. Preferably, the pads are provided on surfaces of the brace which are not patient-specific, as the patient-specific contact surfaces as described above ensure a tight fit even without pads. However, the brace may be designed such that an optimal fit is obtained when the brace is provided with one or more resilient pads. For example, the contact surface may be provided with one or more recesses for receiving a resilient pad. In other embodiments, the brace may not be provided with any pads. This may be preferred for hygienic or other reasons.

In addition or as an alternative to the resilient pads, the inner surface of the brace may be provided with one or more inflatable cushions. The cushions may be progressively inflated upon a decrease of the swelling of the limb, thereby ensuring a progressive tightening of the brace.

In particular embodiments, the brace may be provided with one or more pressure sensing means. This may help in avoiding excessive stresses on the skin, and may help in deciding when to tighten the brace. Suitable pressure sensing means may include, but are not limited electronic pressure sensors, mechanical diaphragms, or a reservoir with liquid which above a specified pressure transfers to another container. The brace may further be provided with an indicator which generates an alarm signal when the pressure is too low or too high. The alarm may be given by an audible, visible or a vibratory method, or by a combination thereof.

The method of the present invention allows for designing patient-specific braces. In particular embodiments, the present method may further comprise the manufacture of the braces which are designed in step (b) as described above. Preferably, the manufacture of the braces involves the use of rapid prototyping techniques such as Additive Manufacturing (AM). Accordingly, in particular embodiments, the present method may further comprise the step of (c) manufacturing the brace designed in step (b) at least partially via additive manufacturing (AM).

AM can be defined as a group of techniques used to fabricate a tangible model of an object typically using three-dimensional (3D) computer aided design (CAD) data of the object. Currently, a multitude of Additive Manufacturing techniques is available, including stereolithography, Selective Laser Sintering, Fused Deposition Modeling, foil-based techniques, etc.

Selective laser sintering uses a high power laser or another focused heat source to sinter or weld small particles of plastic, metal, or ceramic powders into a mass representing the 3-dimensional object to be formed. Fused deposition modeling and related techniques make use of a temporary transition from a solid material to a liquid state, usually due to heating. The material is driven through an extrusion nozzle in a controlled way and deposited in the required place as described among others in U.S. Pat. No. 5.141.680. Foil-based techniques fix coats to one another by means of gluing or photo polymerization or other techniques and cut the object from these coats or polymerize the object. Such a technique is described in U.S. Pat. No. 5.192.539.

Typically AM techniques start from a digital representation of the 3D object to be formed. Generally, the digital representation is sliced into a series of cross-sectional layers which can be overlaid to form the object as a whole. The AM apparatus uses this data for building the object on a layer-by-layer basis. The cross-sectional data representing the layer data of the 3D object may be generated using a computer system and computer aided design and manufacturing (CAD/CAM) software.

In certain embodiments, the patient-specific elements of the brace are manufactured via AM, whereas other elements may be standard elements which are made via other techniques. For example, the patient-specific contact surface(s) may be provided as separate parts, which can be combined with standard first and second portions. This can reduce the time needed for manufacturing the brace. Moreover, this may allow for manufacturing the patient-specific parts and standard parts from different materials. For example, the patient-specific contact surface may be made of a polymer which is compatible with the chosen additive manufacturing technique, whereas the rest of the first and/or second portion may be made of a stronger material such as stainless steel. Other examples of standard elements may include straps, clasps, pads, pressure sensors, etc.

The braces described herein may be manufactured in various materials. In general, any material which is able to withstand normal use forces may be used for manufacturing the braces. Suitable materials include but are not limited to polyamides, polycarbonate, polyamide with additives such as glass or metal particles, polyurethane, metals, alloys and composites such as carbon fiber in an epoxy binder.

Further provided herein is a patient-specific brace for supporting a fractured limb, obtainable by or obtained by the design method described herein. More particularly, the brace may comprises first and second portions having an adjustable position relative to each other so as to adjust the internal dimensions of the brace, wherein the interior of the brace is provided with a patient-specific contact surface which is the mirror image of a corresponding part of the outer surface of a 3D model of the contralateral non-fractured limb.

Further provided herein is a computer program which is configured for carrying out the design method described above. In particular embodiments, computer programs, which, when running on a computer, generate the braces as disclosed herein are provided. In particular embodiments the computer programs are adapted to perform the different steps of the design method described herein. In further embodiments, computer programs comprise software code adapted to perform the steps of the design method described herein. The data processing system or computer program particularly refer to computer aided design and manufacturing systems and programs such as CAD/CAM systems or programs. Said computer programs typically comprise tools for loading images of the contralateral limb, tools for generating a 3D model of said limb based on the images, tools for designing the guiding instrument, and optionally tools for instructing a manufacturing system to manufacture the guiding instrument according to the generated design.

Further provided herein is a method for treating a fracture of a fractured limb, involving the use of a patient-specific fracture brace as described above. Thus, the brace is designed based on a 3D model and/or morphological data of the contralateral (non-fractured) limb. The method is particularly suitable for the treatment of fragility fractures, but may be used for the treatment of other fractures, including those occurring in non-osteoporotic and growing patients.

More particularly, the present method for treating a fracture comprises the steps of:
(i) optionally performing a preliminary reduction of said fracture;
(ii) providing a 3D model of the contralateral limb;
(iii) providing a patient-specific brace for treating said fracture, wherein said brace is designed based on said 3D model provided in step (ii);
(iv) performing (further) reduction of said fracture; and
(v) applying said patient-specific brace to said fractured limb.

The method comprises a step (i) which involves a preliminary reduction of the fracture. This typically involves a first realignment of the limb. After the preliminary reduction, the fractured limb may be provided with a temporary support. Indeed, although the design method described herein may allow for designing and manufacturing the patient-specific brace in a short time, it may be preferred to provide a temporary support to the fractured limb to bridge the time period between steps (i) and (v) of the present method of treating a fracture. Thus, in certain embodiments, a temporary cast or (standard) brace may be applied to the limb after the preliminary reduction of step (i). The temporary cast or brace is then removed from the fractured limb prior to step (iv) or (v).

The method further comprises in a step (ii) providing a 3D model of the contralateral limb.

The details of step (a) of the design method as described above apply, *mutatis mutandis,* to step (ii) of the method of treating a fracture.

The order of steps (i) and (ii) is not critical. Indeed, as the brace is designed based on images of the contralateral limb instead of the fractured limb, the fracture need not be reduced prior to the design of the brace, in contrast with prior art methods of designing fracture braces. Thus, step (i) can be performed prior to step (ii), simultaneously with step (ii), or after step (ii).

Once step (ii) is completed, the 3D model is used for designing and manufacturing a patient-specific brace for supporting the fractured limb in a step (iii). The details of step (b) of the design method as described above apply, *mutatis mutandis,* to step (iii) of the method of treating a fracture. In particular, the brace may be manufactured at least partially via additive manufacturing, as described above

The present method of treating a fracture may further comprise step (iv) which involves performing (further) reduction of the fracture. This is typically performed prior to application of the patient-specific brace as described herein. Step (iv) may be optional when the preliminary reduction performed in step (i) was sufficient. Thus, in certain embodiments, a single reduction step (i) or (iv) may suffice.

The patient-specific brace provided in step (iii) of the present method is applied to the fractured brace in a step (v), optionally after a further reduction in step (iv). The relative position of the first and second portion of the brace is adjusted such that the brace provides sufficient support, without disturbing the blood flow within the limb too much.

As described above, the internal dimensions of the brace are adjustable, such that the brace can be adapted to dimensional changes of the fractured limb. More particularly, fractured limbs initially often exhibit edema. When the edema subsides, the brace can be progressively tightened to anatomically contain the fractured limb, up to bone healing. Accordingly, in particular embodiments the present method of treating a fracture may comprise a further step (vi) of progressive tightening of the brace upon a decrease of swelling of the fractured limb, to maintain pressure on the fractured limb.

## Claims

1. A method of designing a patient-specific brace for positioning on a fractured limb, said method comprising:
(a) providing a three-dimensional (3D) model and/or morphological data of at least a part of a contralateral (i.e. not fractured) limb; and
(b) designing a patient-specific brace for said fractured limb, based on said 3D model and/or morphological data provided in step (a) and assuming symmetry between said fractured limb and said contralateral limb, wherein said brace comprises first and second portions having an adjustable position relative to each other so as to adjust the internal dimensions of said brace.

2. The method according to claim 1, wherein the interior of said brace is provided with a patient-specific contact surface which is the mirror image of a corresponding part of the outer surface of said 3D model.

3. The method according to claim 1 or 2, wherein said first and second portions are coupled to each other via one or more straps.

4. The method according to claim 3, wherein said one or more straps are elastic.

5. The method according to any one of claims 1 to 4, wherein said brace comprises a casing which is at least partially made of a resilient material so as to adjust the internal dimensions of said casing.

6. The method according to any one of claims 1 to 5, wherein the inner surface of said brace is provided with one or more inflatable cushions.

7. The method according to any one of claims 1 to 6, wherein the inner surface of said brace is provided with one or more resilient pads.

8. The method according to any one of claims 1 to 7, wherein said morphological data comprises the length and the perimeter of said contralateral limb.

9. The method according to any one of claims 1 to 8, wherein said brace further comprises one or more pressure sensors.

10. The method according to any one of claims 1 to 9, wherein the fracture of said fractured limb is a wrist fracture or an ankle fracture.

11. The method according to any one of claims 1 to 10, further comprising the step of:
(c) manufacturing said brace at least partially via additive manufacturing.

12. A patient-specific brace obtained by the method according to any one of claims 1 to 11.

13. A patient-specific brace configured to fit a fractured limb, wrist or ankle comprising first and second portions having an adjustable position relative to each other so as to adjust the internal dimensions of said brace, **characterized in that** said patient-specific brace is designed based on the morphology of the contralateral limb, wrist or ankle.

14. The patient-specific brace according to claim 13, wherein said first and second portions are coupled to each other via one or more straps.

15. The patient-specific brace according to claims 13 or 14, wherein said one or more straps are elastic.

16. The patient-specific brace according to any one of claims 13 to 15, wherein said brace comprises a casing which is at least partially made of a resilient material so as to adjust the internal dimensions of said casing.

17. The patient-specific brace according to any one of claims 13 to 16, wherein the inner surface of said brace is provided with one or more inflatable cushions.

18. The patient-specific brace according to any one of claims 13 to 17, wherein the inner surface of said brace is provided with one or more resilient pads.

19. A computer program on a computer-readable storage medium, wherein said program is configured for carrying out the method according to any one of claims 1 to 11.

20. A method of treating a fracture of a fractured limb, said method comprising:
(i) optionally performing a preliminary reduction of said fracture;
(ii) providing a 3D model and/or morphological data of the contralateral (i.e. non-fractured) limb;
(iii) providing a patient-specific brace for treating said fracture, wherein said brace is designed based on said 3D model and/or morphological data provided in step (ii);
(iv) optionally performing further reduction of said fracture; and
(v) applying said patient-specific brace to said fractured limb.

21. The method according to claim 20, further comprising:
(vi) progressive tightening of said brace upon a decrease of swelling of said fractured limb to maintain pressure on said fractured limb.
